(19) **Europäisches Patentamt**
**European Patent Office**
**Office européen des brevets**

(11) **EP 4 776 222 A2**

(12) **EUROPEAN PATENT APPLICATION**

(43) Date of publication:
**15.07.2026 Bulletin 2026/29**

(21) Application number: **26181498.2**

(22) Date of filing: **29.04.2024**

(51) International Patent Classification (IPC):
***G06T 7/00*** *(2017.01)*

(52) Cooperative Patent Classification (CPC):
**A61N 5/1037; A61N 5/1049; G06T 7/0012;**
**G06T 7/0014; G06T 7/248; G06T 7/251;**
**G16H 20/40; G16H 30/40; G16H 50/20;** G06T 7/246

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(62) Document number(s) of the earlier application(s) in
accordance with Art. 76 EPC:
**24723489.1 / 4 661 961**

(71) Applicant: **Brainlab SE**
**81829 München (DE)**

(72) Inventors:
• **BERLINGER, Kajetan**
**80797 München (DE)**
• **FREISLEDERER, Philipp**
**80687 München (DE)**

(74) Representative: **SSM Sandmair**
**Patentanwälte Rechtsanwalt**
**Partnerschaft mbB**
**Joseph-Wild-Straße 20**
**81829 München (DE)**

Remarks:
This application was filed on 28-05-2026 as a
divisional application to the application mentioned
under INID code 62.

(54) **SOFT TISSUE LOCALIZATION IN RADIATION TREATMENT USING SIMILAR MOVEMENT PATTERNS**

(57) Disclosed is a computer-implemented methods of determining the position of an image representation of an anatomical structure having a movement pattern which is similar to a movement pattern of a target structure considered for radiation treatment. The disclosed methods encompass, in one aspect, acquiring a planning computed x-ray tomography of a patient including an image representation of a target structure, such as a tumour, which is subject to movement, reading information describing which describes an anatomical structure which moves together with the tumour in a similar movement pattern, and determining, from the planning computed x-ray tomography a synthesized two-dimensional radiography including an image representation of the tumour and the anatomical structure. The image representation of the anatomical structure can be used as a surrogate, i.e. a proxy, for the image representation of the target structure, i.e. the image representation of the anatomical similarity structure is used for tracking the position of the target structure when matching the tracking reference image with a patient tracking image which shows the live position and/or motion of the anatomical similarity structure but in which the target structure might be of inferior visibility, for example might be occluded. The anatomical similarity structure can be determined using an atlas such as a dynamic atlas or an accordingly configured trained trainable algorithm. The tracking result, i.e. the result of comparing the actual position of the tumour to the planned position of the tumour, can be used for controlling a radiation treatment device.

```
┌─────────────────────────────┐
│            S11              │
│   acquire patient image     │
│           data              │
└─────────────────────────────┘
               │
               ▼
┌─────────────────────────────┐
│            S12              │
│   acquire similarity        │
│      structure data         │
└─────────────────────────────┘
               │
               ▼
┌─────────────────────────────┐
│            S13              │
│  determine tracking         │
│   reference image data      │
└─────────────────────────────┘
```

**Fig. 1**

**Description**

**FIELD OF THE INVENTION**

The present invention relates to computer-implemented methods of determining the position of an image representation of an anatomical structure having a movement pattern which is similar to a movement pattern of a target structure considered for radiation treatment, corresponding computer programs, computer-readable storage media storing such programs and computers executing such programs, as well as a medical system comprising an electronic data storage device and at least one of the aforementioned computers.

**TECHNICAL BACKGROUND**

The localization of a lung tumour in x-ray projection images constitutes a challenge in radiotherapy. First attempts to render only the tumour (i.e. the gross tumour volume) inside a digitally reconstructed radiograph (DRR) were in many cases not successful due to overlapping anatomical structures occluding the target in the x-ray images acquired for target tracking during radiotherapy. The surrogate DRR approach disclosed in US 10,776,959 B2 meant big progress, as after a motion analysis performed, all voxels, i.e. anatomical structures, moving the same way the target does are rendered inside the DRR.

A challenge posed by the surrogate DRR approach is that the amount of respiratory motion occurring in the upper lung lobes - and thus of targets located there - is not as large as the respiratory motion in the lower lung lobes. Thus, finding anatomical structures moving in synchronization with the target at a similarly small movement amplitude becomes difficult. For example, anatomical structures which shall be used as a proxy for the movement of the target may be wrongly determined because they may actually not move but may nevertheless be output as a result due to the small amplitude of the movement of the target. A crop of the CT on the lung lobe of interest in this case fixes this problem, as here the causal connection of movement together with the target is mostly guaranteed.

The present invention has the object of providing improved means for determining a synthesized tracking image for tracking movement of a tumour.

The present invention can be used for radiation treatment procedures e.g. in connection with a system for image-guided radiotherapy such as VERO® and ExacTrac Dynamic®, both products of Brainlab SE.

Aspects of the present invention, examples and exemplary steps and their embodiments are disclosed in the following. Different exemplary features of the invention can be combined in accordance with the invention wherever technically expedient and feasible.

**EXEMPLARY SHORT DESCRIPTION OF THE INVENTION**

In the following, a short description of the specific features of the present invention is given which shall not be understood to limit the invention only to the features or a combination of the features described in this section.

The disclosed methods encompass, in one aspect, acquiring a planning computed x-ray tomography of a patient including an image representation of a target structure, such as a tumour, which is subject to movement, reading information describing which describes an anatomical structure which moves together with the tumour in a similar movement pattern, and determining, from the planning computed x-ray tomography a synthesized two-dimensional radiography (DRR) including an image representation of the tumour and the anatomical structure. The image representation of the anatomical structure can be used as a surrogate, i.e. a proxy, for the image representation of the target structure, i.e. the image representation of the anatomical similarity structure is used for tracking the position of the target structure when matching the tracking reference image with a patient tracking image which shows the live position and/or motion of the anatomical similarity structure but in which the target structure might be of inferior visibility, for example might be occluded. For example, the spinal cord as a target structure comprising a tumour never appears in an x-ray image, but the vertebrae will. The position of the spinal cord is always embedded in the spine and its vertebrae and has a movement pattern if the patient moves which is similar to the movement pattern of the spine and its vertebrae. The spinal cord can be therefore detected in the x-ray image using this approach using the vertebrae as a surrogate, i.e. proxy, for the position of the spinal cord. Source of the knowledge of the similarity of the movement patterns of the spinal cord and the vertebrae in this case can be a static atlas or a trained neural network. The anatomical similarity structure can be determined using an atlas such as a dynamic atlas or an accordingly configured trained trainable algorithm. The tracking result, i.e. the result of comparing the actual position of the tumour to the planned position of the tumour, can be used for controlling a radiation treatment device.

**GENERAL DESCRIPTION OF THE INVENTION**

In this section, a description of the general features of the present invention is given for example by referring to possible embodiments of the invention.

In general, the invention reaches the aforementioned object by providing, in a first aspect, a computer-implemented method of determining a digital image usable for tracking the position of a target structure in radiation treatment. The method according to the first aspect is for example a medical method. The method according to the first aspect comprises executing, on at least one processor of at least one computer, the following exemplary steps which are executed by the at least one processor.

In a (for example first) exemplary step of the method according to the first aspect, patient image data is acquired which describes a digital patient image including an image representation of an anatomical body part of a patient including an image representation of the target structure. The patient image is for example a planning image usable or used for planning radiation treatment such as at least one of radiotherapy and radiosurgery. The target structure is a target which shall be irradiated with a treatment beam of ionizing radiation, for example a tumour. The anatomical body part comprises at least one of soft tissue (for example, an internal organ) and hard tissue (for example, a bony structure or cartilage), for example it is or comprises the lung or another organ or the spine or another bony structure. For example, the patient image is three-dimensional image data, i.e. image data in which positions in the image are defined in three dimensions, such as tomographic image data which has been generated for example by computed x-ray tomography, magnetic resonance tomography or ultrasound tomography. The target structure is associated with a target movement pattern describing a sequence of for example different positions of the target structure. The target structure moves for example due to vital movement such as breathing motion or because the patient is re-positioned or voluntarily moves his body. For example, the patient image data is or as been generated at different points in time, i.e. is time-dependent, and the target movement pattern is derived for example from the time-dependent image described by the patient image data. Specifically, the image describes the position of the target structure at different points in time. Within the framework of this disclosure, the target movement pattern may describe that the target structure remains at the same position for all of the different points in time, i.e. remains inert. In other words, the target movement pattern may describe that the target structure attains different positions each associated with one of the different points in time or that the target structure does not move throughout the different points in time.

In a (for example second) exemplary step of the method according to the first aspect, similarity structure data is acquired which describes an anatomical structure called anatomical similarity structure included in the anatomical body part. The anatomical similarity structure is associated with a similar movement pattern which is similar to the target movement pattern. For example, the anatomical similarity structure moves for the same reason as the target structure, even though possibly in a different time- and/or position-wise pattern which, however, fulfils a condition for similarity to the target movement pattern. For example, the anatomical similarity structure moves at least substantially when, for example together with, the target structure moves. For example, the similarity structure data describes a data object including and/or describing the anatomical similarity structure. The similar movement pattern describes a sequence of for example different positions of the similarity structure, wherein the patient image includes an image representation of the anatomical similarity structure. Within the framework of this disclosure, the similar movement pattern may describe that the anatomical similarity structure remains at the same position for all of the different points in time, i.e. remains inert. In other words, the similarity movement pattern may describe that the anatomical similarity structure attains different positions each associated with one of the different points in time or that the target structure does not move throughout the different points in time. The similar movement pattern is similar to the target movement pattern to a predetermined degree. For example, the similarity is or has been determined by comparing the shape of the polyline representing the movement, i.e. the trajectory, of the target structure to the shape of the polyline representing the movement, i.e. the trajectory, of the anatomical similarity structure, for example by determining a distance representing the predetermined degree such as an average Euclidean distance between nodes of the polyline representing the movement, i.e. the trajectory, of the target structure on the one hand and nodes of the polyline representing the movement, i.e. the trajectory, of the anatomical similarity structure on the other hand. For example, the trajectory, for example the shape of the trajectory, of the target structure serves as a reference to which the trajectory, for example the shape of the trajectory, of the anatomical similarity structure and, in the case of a plurality of anatomical similarity structures being considered, the trajectory, for example the shape of the trajectory, of each of the anatomical similarity structures is compared. If the determined value of the distance is greater than or equal to the predetermined degree, the trajectory, for example the shape of the trajectory, of the target structure and the trajectory, for example the shape of the trajectory, of the anatomical similarity structure, are determined to be similar. For example, the distance between the trajectories is determined in a common reference system in which the starting points of the trajectories to be compared are superimposed.

An exemplary pseudocode for calculating the degree of similarity reads as follows:

```
double mean = 0.0;

Vector3D offset = refTraj[0] – traj1[0];


for (int i = 0; i < refTraj.size(); i++)

    mean += Euclidian_Distance3D(refTraj[i], traj1[i] + offset);

mean = mean / refTraj.size();
```

Here, mean is the average value of the distance between the trajectories which are a reference trajectory refTraj and a trajectory traj1 to which the reference trajectory shall be compared. The variable mean has been calculated from individual Euclidian distances between corresponding nodes of the trajectories by applying the function Euclidean_Distance3D in three dimensions, offset is the difference in starting position of the two trajectories (which will be zero in case the trajectories have been superimposed), i is the counting variable for positions along the trajectories (i.e. the numbering of nodes of the trajectories)

In a (for example third) exemplary step of the method according to the first aspect, tracking reference image data is determined based on the patient image data and the similarity structure data. The tracking reference image data describes a digital tracking reference image including an image representation of the target structure and an image representation of the anatomical similarity structure. The tracking reference image is determined by extracting, from the patient image, the image representation of the target structure and the image representation of the similarity structure from the patient image. For example, the tracking reference image is determined by extracting, from the patient image, only image representation of the target structure and the image representation of the anatomical similarity structure from the patient image. For example, the image representations of the target structure and the anatomical similarity structure are defined by each a data object including the respective image representation, and the tracking reference image is determined by extracting these data objects from the patient image. For example, the tracking reference image is determined by clipping, from the patient image, the image representation, for example a data object including the image representation, of the target structure and the image representation, for example a data object including the image representation, of the anatomical similarity structure from the patient image. For example, the image constituents of the tracking reference image comprise only the clipped image contents, i.e. image representations of the target structure and the anatomical similarity structure. Specifically, the image contents of the tracking reference image is defined only by data objects describing the target structure and the anatomical similarity structure. For example, the image representation of the target structure is included in a data object in the patient image called patient target object and the image representation of the anatomical similarity structure is included in a data object in the patient image called patient similarity object, and the tracking image data is determined by extracting, for example clipping, the patient target object and the similarity object from the patient image. The image representation of the anatomical similarity structure is for example used as a surrogate, i.e. a proxy, for the image representation of the target structure, i.e. the image representation of the anatomical similarity structure is for example used for tracking the position of the target structure when matching the tracking reference image with a patient tracking image which shows the live position and/or motion of the anatomical similarity structure but in which the target structure might be of inferior visibility, for example might be occluded, for example due to low attenuation of the imaging (x-ray) radiation by the target structure such as in the case of the target structure comprising or consisting of soft tissue. In this context, it is assumed that the relative position between the target structure and the anatomical similarity structure is predetermined, for example known from the patient image, and has not changed in the patient tracking image, i.e. that the relative position between the target structure and the anatomical similarity structure is at least substantially the same in the patient image and/or the tracking reference image on the one hand and the patient tracking image on the other hand. The tracking reference image describes a planned position of the image representation of the anatomical similarity structure, i.e. assumingly of the anatomical similarity structure, and the patient tracking image describes the actual position of the image representation of the anatomical similarity structure, i.e. assumingly of the anatomical similarity structure. For example, a result of comparison between these two positions, i.e. a determination whether the anatomical similarity structure and therefore assumingly the target structure is at the planned position, is used as a decision criterion for determining control data for controlling for controlling a radiation treatment device, i.e. issue of a treatment beam, or as a decision criterion for controlling a radiation treatment device, i.e. issue of a treatment beam.

For example, positions in the tracking reference image are defined in the reference system used for defining positions in the patient image, i.e. positions in both images are defined in the same reference system. For example, the tracking reference image is determined by projecting the extracted image representation and/or data objects into two dimensions to generate a synthesized two-dimensional medical image such as a synthesized radiography (i.e. a digitally reconstructed radiograph) of the target structure and the anatomical similarity structure.

In an example of the method according to the first aspect, atlas data is acquired which describes a digital model, for example a three-dimensional digital model, of the anatomical body part. The atlas data comprises for example static atlas data describing data objects, called atlas objects, which define a spatial property, for example at least one of geometry and position, of constituents, for example the constituents, of the anatomical body part. Furthermore, target position data is acquired which describes a position of the target structure in the patient image. Then, target object data determined based on the target position data and the atlas data, for example by matching the patient image data with the atlas data (e.g. by applying an image fusion algorithm to the patient image data and the atlas data, i.e. by fusing the two datasets). The target object data describes a data object in the atlas data including the target structure (i.e. a representation in the digital model of the target structure), called atlas target object. The similarity structure data is determined based on the target object data and the patient image data, for example by segmenting, from the patient image, a data object corresponding to the atlas target object and associated with the target movement pattern and determining a data object having the similarity movement pattern, i.e. a movement pattern which similar to the target movement pattern, and by determining the anatomical similarity structure to be the data object determined int this way.

For example, the atlas data comprises (for example in addition to the static atlas data) dynamic atlas data describing a dynamic property, for example a change of the spatial property of the associated, for example for each of, the atlas objects, i.e. the data objects described by the atlas data. For example, the dynamic property describes at least one trajectory defining a time-dependent position of the associated atlas object or of a point with a known spatial relationship to the associated atlas object, such as a centre of the associated atlas object or a point on the outline of the associated atlas object. For example, the dynamic property of the target data object described by the dynamic atlas data is the target movement pattern and the dynamic property of the similarity data object described by the dynamic atlas data is the similarity movement pattern. In this example, target property data is determined based on the target object data. The target property data describes the dynamic property associated with the target data object, called target property. Furthermore, similarity object data describing a data object, called atlas similarity object, is determined based on the target property data and the atlas data. The atlas similarity object is associated with a dynamic property, called similarity property, which is similar to the target property. The similarity property is defined to be similar to the target property if it fulfils a predetermined degree of similarity. For example, the similarity is defined as a value of a correlation between the dynamic properties, e.g. movement patterns such as trajectories, of (i.e. associated with) different atlas objects such as the atlas similarity object and the atlas target object. The similarity structure data is then determined based on the similarity object data and the patient image data, for example by searching the patient image, for example a segmentation of the patient image generated by matching the patient image with the model described by the atlas data, for a data object corresponding to the atlas target object.

A trajectory of an atlas object is for example described by global vectors including all values of the vector $\vec{p}(t)$ (e.g. $\overrightarrow{p_{x,y}}(t)$ or $\overrightarrow{p_{x,y,z}}(t)$) at several points in time (e.g. all point in time for which data is available), where $\vec{p}(t)$ is the position $p$ of the associated data object as a function of time $t$. $p$ can be defined in two (e.g. for $x$ and $y$) or three (e.g. for $x$, $y$ and $z$) dimensions. For example, the global vector $\overrightarrow{p_{glob}}$ comprises all vectors $\overrightarrow{p_{x,y,z}}(t)$ for (n+1) points in time: $\overrightarrow{p_{x,y,z}}(t_0)$, $\overrightarrow{p_{x,y,z}}(t_1)$, ..., $\overrightarrow{p_{x,y,z}}(t_n)$. For example, all vector values are stored in the global vector as follows:

$$\overrightarrow{p_{glob}} = \{p_x(t_0), p_y(t_0), p_z(t_0), p_x(t_1), p_y(t_1), p_z(t_1), \dots, p_x(t_n), p_y(t_n), p_z(t_n)\}.$$

The dynamic atlas data describes for example only closed trajectories which alternatively or additionally exhibit a periodic temporal behavior.

To determine the correlation between two trajectories, for example a global vector $\overrightarrow{p_{glob}}$ describing the respective trajectory can be used. For example, a global vector $\overrightarrow{p_{glob,1}}$ describing a first trajectory and a global vector $\overrightarrow{p_{glob,2}}$ describing a second trajectory can be used to determine the correlation between the first and the second trajectory.

The correlation between the first trajectory described by all values of $\vec{p}(t)$ and the second trajectory described by all values of $\vec{q}(t)$ can for example be determined as follows:

$$Corr = \frac{\int_{t=t_0}^{t=t_n}(\vec{p}(t) - \vec{p}(t_0))(\vec{q}(t) - \vec{q}(t_0)dt}{\sqrt{\int_{t=t_0}^{t=t_n}(\vec{p}(t) - \vec{p}(t_0))(\vec{p}(t) - \vec{p}(t_0)dt}\sqrt{\int_{t=t_0}^{t=t_n}(\vec{q}(t) - \vec{q}(t_0))(\vec{q}(t) - \vec{q}(t_0)dt}} .$$

Alternatively or additionally, a component-wise or weighted correlation may be used. For example, all correlations between all trajectories $(\vec{p}(t),\ \vec{q}(t),\ \vec{r}(t),\ \vec{s}(t),\ ...)$ of different spatial atlas elements (P, Q, R, S, ...) are calculated as a matrix (CM).

The direction Dir of each trajectory, for example of the trajectory described by all values of $\vec{p}(t)$ of spatial atlas element P, can also be calculated, for example as follows:

$$Dir = \frac{\vec{p}(t) - \vec{p}(t_0)}{\sqrt{\int_{t=t_0}^{t=t_n}(\vec{p}(t) - \vec{p}(t_0))(\vec{p}(t) - \vec{p}(t_0)dt}}$$

or

$$Dir = \frac{\vec{p}(t_0)}{|\vec{p}(t_0)|} .$$

The direction *Dir* is for example be averaged and/or normalized and associated to and/or stored for the corresponding atlas object. The direction *Dir* is for example not a scalar and can therefore for example be back-transformed into the model described by the atlas data before averaging and/or normalizing.

For example, the dynamic property classified according to patient type. The available patient types for example include one or more of patients with a certain disease, patients with a certain age, patients with a certain anatomical property (obese, missing organs, deformed organs etc.), or patients with a certain gender. For example, the information on the at least one dynamic property linked to the atlas object is classified (i.e. split up) according to different patient types depending on information of individuals from different patient types, for example information on the dynamic property of the anatomic body part of individuals from different patient types, for example individuals belonging to different patient types used upon creating and/or improving the model described by the atlas data. In one example, the information on the dynamic property linked to an atlas object is classified by classifying (e.g. splitting up) the associated trajectory according to patient types.

For example, the dynamic property is used as a constraint, i.e. boundary condition, for matching the digital model with the patient image, for example to determine at least one of the similarity object data and the similarity structure data. A constraint for the matching is for example that a certain atlas object is associated with a certain dynamic property which the corresponding data object in the patient image must also exhibit to a predetermined degree or to which the corresponding data object in the patient image must not be in contradiction. For example, the constraint is defined as the associated dynamic property describing a maximum change of geometry and/or position of the respective atlas object in absolute terms or relative to other elements (e.g. maximum distance to another element during positional change caused by vital movements), which should not be violated by the matching (e.g. by image fusion). For example, the constraint is defined as the corresponding data object in the patient image having a dynamic property which lies within a certain range defined by the dynamic property of the corresponding atlas object and fulfills a predetermined range threshold. For example, the location of a possibly corresponding data object in the patient image is constrained to lie in a certain area. For example, the positional change of a possibly corresponding data object in the patient image be constrained to a certain amplitude, phase, direction, deviation from a given trajectory or else. For example, the volumetric change and/or deformation of a possibly corresponding data object in the patient image is constrained to a certain maximum value, phase, deviation from a given rate or absolute value of (volumetric or deformational) change or else. In other words, the static atlas data may be used for the matching, but in the dynamic atlas data is for example used in addition to the static atlas data for the matching in the form of a constraint, e.g. a constraint limiting the possible positions of a data object in the patient image corresponding to the respective atlas object.

In a second aspect, the invention relates to a computer-implemented method of training a trainable, for example a learning algorithm such as a machine learning algorithm, to determine anatomical structures having a similar movement pattern. The method according to the second aspect is for example a medical method. The method comprises executing, on at least one processor of at least one computer, the following exemplary steps which are executed by the at least one processor. In an example, the trainable algorithm comprises or consists of a convolutional neural network.

In a (for example first) exemplary step of the method according to the second aspect, image training data is acquired which describes at least two digital for example three-dimensional training images of an anatomical body part including a for example segmented training target data object including an image representation of an anatomical body part of a patient. The image representation of the anatomical body part includes an image representation of a target structure. The target structure is associated with a training target movement pattern describing a sequence of for example different positions of the target structure. Within the framework of this disclosure, the training target movement pattern may describe that the target structure remains at the same position for all of the different points in time, i.e. remains inert. In other words, the training target movement pattern may describe that the target structure attains different positions each associated with one of the different points in time or that the target structure does not move throughout the different points in time. The training images have been generated at different points in time and describe different positions of the of the target structure. The training images include at least one similarity training data object which is for example different from the training target data objects and includes a for example segmented image representation of an anatomical structure included in the anatomical body part which is for example at least partially different from the target structure, called training similarity structure. The training similarity structure is associated with a training similar movement pattern which is similar to the target movement pattern. The training similar movement pattern describes a sequence of for example different positions of the training similarity structure. Within the framework of this disclosure, the training similar movement pattern may describe that the target structure remains at the same position for all of the different points in time, i.e. remains inert. In other words, the training similar movement pattern may describe that the target structure attains different positions each associated with one of the different points in time or that the target structure does not move throughout the different points in time. The training similar movement pattern is similar to the training target movement pattern to a predetermined degree. For example, the similarity is or has been determined by comparing the shape of the polyline representing the movement, i.e. the trajectory, of the target structure to the shape of the polyline representing the movement, i.e. the trajectory, of the training similarity structure, for example by determining a distance representing the predetermined degree such as an average Euclidean distance between nodes of the polyline representing the movement, i.e. the trajectory, of the target structure on the one hand and nodes of the polyline representing the movement, i.e. the trajectory, of the training similarity structure on the other hand. For example, the trajectory, for example the shape of the trajectory, of the target structure serves as a reference to which the trajectory, for example the shape of the trajectory, of the training similarity structure and, in the case of a plurality of training similarity structures being considered, the trajectory, for example the shape of the trajectory, of each of the training similarity structures is compared. If the determined value of the distance is greater than or equal to the predetermined degree, the trajectory, for example the shape of the trajectory, of the target structure and the trajectory, for example the shape of the trajectory, of the training similarity structure, are determined to be similar. For example, the distance between the trajectories is determined in a common reference system in which the starting points of the trajectories to be compared are superimposed. Information defining the training target movement pattern and the training similar movement pattern is also acquired by the method according to the second aspect.

In a (for example second) exemplary step of the method according to the second aspect, similarity structure data is determined which describes model parameters of the trainable algorithm for establishing a relation between the target structure and the training similarity structure, wherein the relation describes whether, for example that, the training similar movement pattern is similar to the training target movement pattern and is determined by inputting the image training data into a function, specifically a function of the trainable algorithm, which establishes the relation. For example, the model parameters of the trainable algorithm define the trainable parameters, for example weights, of the trainable algorithm. Thereby, the trainable algorithm is trained, i.e. taught, to determine that a difference between the position of the target structure and the position of the training similarity structure is considered to be similar in comparison between different ones of the training images.

In an example of the method according to the second aspect, similarity training data is acquired which describes whether, for example that, the training target movement pattern is similar to the training similar movement pattern. The similarity structure data is then determined by inputting the similarity training data into the function. Thereby, the labels describing the similarity are used as additional training information.

In an example of the method according to the second aspect, the similarity structure data is determined by determining whether, for example that, the relation fulfils a predetermined criterion which defines that the training similar movement pattern is similar to the training target movement pattern, for example by comparing the relationship to the predetermined criterion and determining that the relationship fulfils the predetermined criterion. In this example, the similarity is determined, for example calculated, separately from, for example after, performing pattern recognition by the trainable algorithm.

In an example of the method according to the second aspect, the medical image training data is patient image data or has been generated from atlas data describing a digital for example three-dimensional model of the anatomical body part.

In an example of the method according to the first aspect, the similarity structure data is or has been determined by inputting the patient image data into a trainable algorithm configured to determine the similarity structure data, for example by executing the trained trainable algorithm on the patient image data. The trainable algorithm has been configured, for example trained, by executing the method according to the second aspect.

In a third aspect, the invention relates to a computer-implemented method of determining control data for controlling the operation of a radiation treatment device. The method according to the third aspect is for example a medical method. The method according to the third aspect comprises executing, on at least one processor of at least one computer, the following exemplary steps which are executed by the at least one processor.

In a (for example first) exemplary step of the method according to the third aspect, the method according to first or second aspect is executed.

In a (for example second) exemplary step of the method according to the third aspect, tracking patient image data is acquired which describes a digital patient tracking image, for example a two-dimensional image such as a radiography, including an image representation of the anatomical similarity structure generated by imaging the anatomical body part.

In a (for example third) exemplary step of the method according to the third aspect, control data for controlling the operation of a radiation treatment device is determined based on the tracking reference image data and the tracking patient image data. The control data describes for example one control signal for controlling the operation of the radiation treatment device based on the tracking reference image data and the tracking patient image data. For this purpose, at least one two-dimensional projection of the tracking reference image, for a example a synthesized radiography such as a digitally reconstructed radiograph is generated with the imaging parameters, for example from the perspective, relative to the anatomical similarity structure used to generate the patient tracking image. Positions in the patient tracking image and the tracking reference image are defined in a common reference system or at least a spatial transformation between positions in the tracking reference image and the patient tracking image is known. The position of the image representation of the anatomical similarity structure in the patient tracking image is compared to the position of the anatomical similarity structure in the tracking reference image, which is for example a planned position. If the comparison leads to a result fulfilling a predetermined condition, a corresponding control signal is generated. For example, the tracking reference image and the tracking patient image are spatially comparable, for example positions in the tracking reference image and the tracking patient image are defined in a common reference system or related to each other by a known spatial transformation, for example a mapping function, and the control data is determined by comparing the position of the image representation of the anatomical similarity structure in the tracking reference image to the position of the image representation of the anatomical similarity structure in the tracking patient image for example using a known image matching algorithm such as image fusion.

An example of the method according to the third aspect encompasses controlling the radiation treatment device based on the control data, for example by executing the control data. Execution of the control data comprises for example controlling at least one of the position of the treatment beam to be issued by the radiation treatment device and issue of the treatment beam by the radiation treatment device.

In a fourth aspect, the invention is directed to a computer program comprising instructions which, when the program is executed by at least one computer, causes the at least one computer to carry out method according to the first, second or third aspect. The invention may alternatively or additionally relate to a (physical, for example electrical, for example technically generated) signal wave, for example a digital signal wave, such as an electromagnetic carrier wave carrying information which represents the program, for example the aforementioned program, which for example comprises code means which are adapted to perform any or all of the steps of the method according to the first aspect. The signal wave is in one example a data carrier signal carrying the aforementioned computer program. A computer program stored on a disc is a data file, and when the file is read out and transmitted it becomes a data stream for example in the form of a (physical, for example electrical, for example technically generated) signal. The signal can be implemented as the signal wave, for example as the electromagnetic carrier wave which is described herein. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The invention according to the second aspect therefore may alternatively or additionally relate to a data stream representative of the aforementioned program, i.e. comprising the program. In examples of the fourth aspect, the invention relates to a data carrier signal carrying the aforementioned program, and/ or a data carrier signal carrying data defining the model parameters and the architecture of a trainable algorithm which has been trained by executing the method according to the second aspect, and/or a data stream which carries the aforementioned program, and/or a data stream which carries data defining the model parameters and the architecture of a learning algorithm which has been trained by executing the method according to the second aspect.

In a fifth aspect, the invention is directed to a computer-readable storage medium on which the program according to the second aspect is stored. The program storage medium is for example non-transitory. In an example of the fifth aspect, the invention relates to a program storage medium on which data defining the model parameters and the architecture of a trainable algorithm which has been trained by executing the method according to the second aspect are stored.

In a sixth aspect, the invention is directed to at least one computer (for example, a computer), comprising at least one processor (for example, a processor), wherein the program according to the second aspect is executed by the processor, or wherein the at least one computer comprises the computer-readable storage medium according to the third aspect.

In a seventh aspect, the invention is directed to a system for planning or conducting radiation treatment, comprising:

    a) the at least one computer according to the sixth aspect;
    b) at least one electronic data storage device storing the patient image data; and
    c) the program storage medium according to the preceding claim,

wherein the at least one computer is operably coupled to the at least one electronic data storage device for acquiring, from the at least one electronic data storage device, the patient image data, and for storing, in the at least one electronic data storage device, at least the tracking reference image data.

In an example, the system according to seventh aspect comprises a radiation treatment device comprising a treatment beam source and a patient support unit (such as at least one of a patient bed or a headrest). The at least one computer is then operably coupled to the radiation treatment device for issuing a control signal to the radiation treatment device for controlling the operation of the radiation treatment device on the basis of the control data determined by executing the method according to the third aspect.

For example, the disclosed method is not a method for treatment of the human or animal body by surgery or therapy. For example, the invention does not involve or in particular comprise or encompass an invasive step which would represent a substantial physical interference with the body requiring professional medical expertise to be carried out and entailing a substantial health risk even when carried out with the required professional care and expertise.

For example, the invention does not comprise a step of irradiating a patient with treatment radiation. Rather, the invention is directed to data processing, for example image analysis and generation and determining of control data. More particularly, the invention does not involve or in particular comprise or encompass any surgical or therapeutic activity.

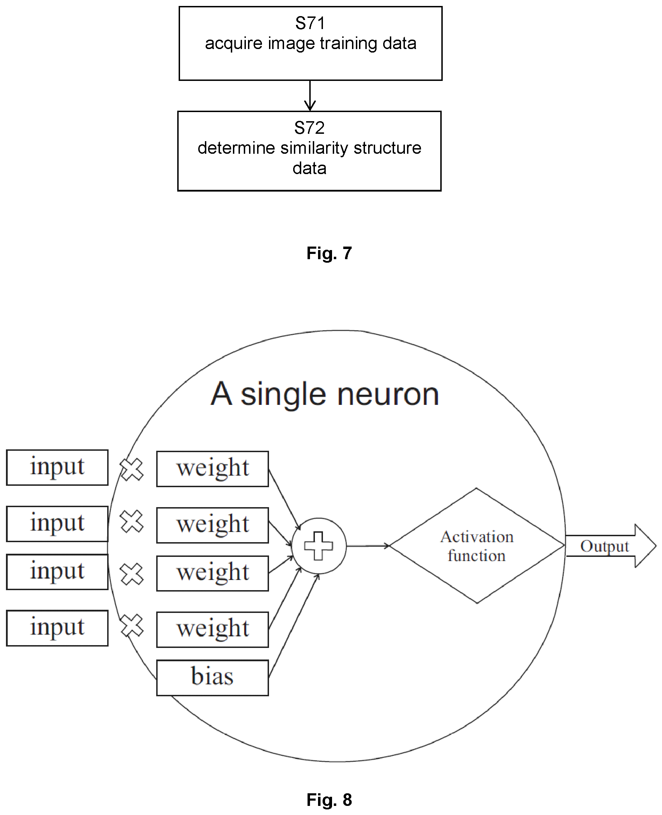

In the following, an explanation of convolutional neural networks as an example of the machine learning algorithm to be used with the disclosed invention is provided with reference to Fig. 8.

Convolutional networks, also known as convolutional neural networks, or CNNs, are an example of neural networks for processing data that has a known grid-like topology. Examples include time-series data, which can be thought of as a 1-D grid taking samples at regular time intervals, and image data, which can be thought of as a 2-D or 3-D grid of pixels. The name "convolutional neural network" indicates that the network employs the mathematical operation of convolution. Convolution is a linear operation. Convolutional networks are simply neural networks that use convolution in place of general matrix multiplication in at least one of their layers. There are several variants on the convolution function that are widely used in practice for neural networks. In general, the operation used in a convolutional neural network does not correspond precisely to the definition of convolution as used in other fields, such as engineering or pure mathematics.

The main component of convolutional neural networks are artificial neurons. Fig. 8 is an example of a single neuron depicted. The node in the middle represents a neuron, which takes all inputs ($x_1$, ..., $x_n$) and multiplies them with their specific weights ($w_1$, ..., $w_n$). The importance of the input depends on the value of its weight. The addition of these computed values is called weighted sum which will be inserted into an activation function. The weighted sum z is defined as:

$$z = \sum_{i=0}^{n} x_i \cdot w_i \qquad\qquad (1)$$

The bias b is an input-independent value which modifies the boundaries of the threshold. The resulting value is processed by an activation function which decides whether the input will be transferred to the next neuron.

A CNN usually takes an order 1 or 3 tensor as its input, e.g., an image with H rows, W columns, and 3 channels (R, G, B colour channels). Higher order tensor inputs, however, can be handled by CNN in a similar fashion. The input then sequentially goes through a series of processing. One processing step is usually called a layer, which could be a convolution layer, a pooling layer, a normalization layer, a fully connected layer, a loss layer, etc. Details of the layers are described in the sections below.

$$x^1 \rightarrow \boxed{w^1} \rightarrow x^2 \rightarrow \cdots \rightarrow x^{L-1} \rightarrow \boxed{w^{L-1}} \rightarrow x^L \rightarrow \boxed{w^L} \rightarrow z \qquad (2)$$

The above equation 2 illustrates how a CNN runs layer by layer in a forward pass. The input is $x^1$, usually an image (order 1 or 3 tensor). We note the parameters involved in the processing of the first layer collectively as a tensor $w^i$. The

output of the first layer is $x^2$, which also acts as the input to the second layer processing. This processing proceeds until processing of all layers in the CNN has been finished, which outputs $x^L$. One additional layer, however, is added for backward error propagation, a method that leans good parameter values in the CNN. Suppose the problem at hand is an image classification problem with C classes. A commonly used strategy is to output $x^L$ as a C-dimensional vector, the i-th entry of which encodes the prediction (posterior probability that $x^1$ comes from the i-th class). To make $x^L$ a probability mass function, we can set the processing in the (L - 1)-th layer as a softmax transformation of $x^{L-1}$. In other applications, the output $x^L$ may have other forms and interpretations. The last layer is a loss layer. Let us suppose t is the corresponding target (ground-truth) value for the input $x^1$, then a cost or loss function can be used to measure the discrepancy between the CNN prediction $x^L$ and the target t. Note that some layers may not have any parameters, that is, $w^i$ may be empty for some i.

In an example of a CNN, ReLu is used as an activation function for the convolutional layers and the softmax activation function provides information in order to give an classification output. The following sections will explain the purpose of the most important layers.

An input image is input to a feature learning section of a layer comprising convolution and ReLu, followed by a layer comprising pooling, which is followed by further pairwise repetitions of layers of convolution and ReLu and of pooling. The output of the feature learning section is input to a classification section which comprises layers directed to flattening, fully connecting and softmaxing.

In a convolutional layer, multiple convolution kernels are usually used. Assuming D kernels are used and each kernel is of spatial span H x W, we denote all the kernels as f. f is an order 4 tensor in $\mathbb{R}^{H \times W \times D^l \times D}$. Similarly, we use index variables $0 \leq i < H$, $0 \leq j < W$, $0 \leq d^l < D^l$ and $0 \leq d < D$ to pinpoint a specific element in the kernels. Also note that the set of kernels f refers to the same object as the notation $w^L$ above. We change the notation a bit to simplify the derivation. It is also clear that even if the mini-batch strategy is used, the kernels remain unchanged.

The spatial extent of the output is smaller than that of the input so long as the convolution kernel is larger than 1 x 1. Sometimes we need the input and output images to have the same height and width, and a simple padding trick can be used

For every channel of the input, if we pad (i.e., insert) $\left\lfloor \frac{H-1}{2} \right\rfloor$ rows above the first row and $\left\lfloor \frac{H}{2} \right\rfloor$ rows below the last row, and paid $\left\lfloor \frac{W-1}{2} \right\rfloor$ columns the left of the first column and $\left\lfloor \frac{W}{2} \right\rfloor$ columns to the right of the last column of the input, the convolution output will be $H^l \times W^l \times D$ in size, i.e. having the same spatial extent as the input. $\lfloor * \rfloor$ is the floor function. Elements of the padded rows and columns are usually set to 0, but other values are also possible.

Stride is another important concept in convolution. A kernel is convolved with the input at every possible spatial location, which corresponds to the stride s = 1. However, if s > 1, every movement of the kernel skip s - 1 pixel locations (i.e., the convolution is performed once every s pixel both horizontally and vertically).

In this section, we consider the simple case when the stride is 1 and no padding is used. Hence, we have y (or $x^{l+1}$) in $\mathbb{R}^{H^{l+1} \times W^{l+1} \times D^{l+1}}$, with $H^{l+1} = H^l - H + 1$, $W^{l+1} = W^l - W + 1$, and $D^{l+1} = D$. In precise mathematics, the convolution procedure can be expressed as an equation:

$$y_{i^{l+1},j^{l+1},d} = \sum_{i=0}^{H} \sum_{j=0}^{W} \sum_{d^l=0}^{D^l} f_{i,j,d^l,d} \times x^l_{i^{l+1}+i,j^{l+1}+j,d^l} \qquad (3)$$

Equation 3 is repeated for all $0 \leq d \leq D = D^{l+1}$, and for any spatial location $(i^{l+1}, j^{l+1})$ satisfying $0 \leq i^{l+1} < H^l - H + 1 = H^{l+1}$, $0 \leq j^{l+1} < W^l - W + 1 = W^{l+1}$. In this equation, $x^l_{i^{l+1}+i,j^{l+1}+j,d^l}$ refers to the element of $x^l$ indexed by the triplet $(i^{l+1}+i, j^{l+1}+j, d^l)$. A bias term $b_d$ is usually added to $y_{i^{l+1},j^{l+1},d}$. We omit this term in this note for clearer presentation.

A pooling function replaces the output of the net at a certain location with a summary statistic of the nearby outputs. For example, a max pooling operation reports the maximum output within a rectangular neighbourhood of a table. Other popular pooling functions include the average of a rectangular neighbourhood, the $L_2$ norm of a rectangular neighbourhood, or a weighted average based on the distance from the central pixel. In all cases, pooling helps to make the representation approximately invariant to small translations of the input. Invariance to translation means that if we translate the input by a small amount, the values of the pooled outputs do not change.

Because pooling summarizes the responses over a whole neighbourhood, it is possible to use fewer pooling units than detector units, by reporting summary statistics for pooling regions spaced k pixels apart rather than one pixel apart. This improves the computational efficiency of the network because the next layer has roughly k times fewer inputs to process.

Suppose all the parameters of a CNN model $w^1, ..., w^{L-1}$ have been learned, then we are ready to use this model for prediction. Prediction only involves running the CNN model forward, i.e., in the direction of the arrows in equation 1. Take the image classification problem as an example. Starting from the input $x^1$, we make it pass the processing of the first layer (the box with parameters w'), and get $x^2$. In turn, $x^2$ is passed into the second layer, etc. Finally, we receive $x^l \in \mathbb{R}^C$, which estimates the posterior probabilities of $x^1$ belonging to the C categories. We can output the CNN prediction as:

$$arg\,_i\,max\,x_i^L \qquad (4)$$

Now, the problem is: how do we learn the model parameters?

As in many other learning systems, the parameters of a CNN model are optimized to minimize the loss z, i.e. we want the prediction of a CNN model to match the ground-truth labels. Suppose one training example $x^1$ is given for training such parameters. The training process involves running the CNN network in both directions. We first run the network in the forward pass to get $x^L$ to achieve a prediction using the current CNN parameters. Instead of outputting a prediction, we need to compare the prediction with the target t corresponding to $x^1$, i.e. continue running the forward pass till the last loss layer. Finally, we achieve a loss z. The loss z is then a supervision signal, guiding how the parameters of the model should be modified (updated).

There exist several algorithms for optimizing a loss function and CNNs are not limited to a specific one. An example algorithm is called Stochastic Gradient Descent (SGD). This means the parameters are updated by using the gradient estimated from a (usually) small subset of training examples.

$$w^i \leftarrow w^i - \eta\frac{\delta z}{\delta w^i} \qquad (5)$$

In equation 5, the $\leftarrow$ - sign implicitly indicates that the parameters $w^i$ (of the i-layer) are updated from time t to t + 1. If a time index t is explicitly used, this equation will look like

$$(w^i)^{t+1} = (w^i)^t - \eta\frac{\delta z}{\delta (w^i)^t} \qquad (6)$$

In equation 6, the partial derivative $\frac{\delta z}{\delta w^i}$ measures the rate of increase of z with respect to the changes in different dimensions of $w^i$. This partial derivative vector is called the gradient in mathematical optimization. Hence, in a small local region around the current value of $w^i$, to move $w^i$ in the direction determined by the gradient will increase the objective value z. In order to minimize the loss function, we should update $w^i$ along the opposite direction of the gradient. This updating rule is called the gradient descent.

If we move too far in the negative gradient direction, however, the loss function may increase. Hence, in every update we only change the parameters by a small proportion of the negative gradient, controlled by $\eta$ (the learning rate). $\eta$ > 0 is usually set to a small number (e.g., $\eta$ = 0.001). One update based on $x^1$ will make the loss smaller for this particular training example if the learning rate is not too large. However, it is very possible that it will make the loss of some other training examples become larger. Hence, we need to update the parameters using all training examples. When all training examples have been used to update the parameters, we nay one epoch has been processed. One epoch will in general reduce the average loss on the training set until the learning system overfits the training data. Hence, we can repeat the gradient descent updating epochs and terminate at some point to obtain the CNN parameters (e.g., we can terminate when the average loss on a validation set increases).

The last layer's partial derivatives are easy to compute. Because $x^L$ is connected to z directly under the control of parameters $w^L$, it is easy to compute $\frac{\delta z}{\delta w^L}$. This step is only needed when $w^L$ is not empty. In the same spirit, it is also easy to compute $\frac{\delta z}{\delta x^L}$. For example, if the squared $L_2$ loss is used, we have an empty $\frac{\delta z}{\delta w^L}$, and $\frac{\delta z}{\delta x^L} = x^L - t$.

In fact, for every layer, we compute two sets of gradients: the partial derivatives of z with respect to the layer parameters $w^i$, and that layer's input xi. The term $\frac{\delta z}{\delta w^i}$, as seen in Equation 4, can be used to update the current (i-th) layer's parameters. The term $\frac{\delta z}{\delta x^i}$ can be used to update parameters backwards, e.g., to the (i-1)-th layer. An intuitive

explanation is: $x^i$ is the output of the (i-1)-th layer and $\frac{\delta z}{\delta x^i}$ is how $x^i$ should be changed to reduce the loss function. Hence, we could view $\frac{\delta z}{\delta x^i}$ as the part of the "error" supervision information propagated from z backward till the current layer, in a layer by layer fashion. Thus, we can continue the back propagation process, and use $\frac{\delta z}{\delta x^i}$ to propagate the errors backward to the (i-1)-th layer. This layer-by-layer backward updating procedure makes learning a CNN much easier.

Take the i-th layer as an example. When we update the i-th layer, the back propagation process for the (i+1)-th layer must have been finished. That is, we already computed the terms $\frac{\delta z}{\delta w^{i+1}}$ and $\frac{\delta z}{\delta x^{i+1}}$. Both are stored in memory and ready for use. Now our task is to compute $\frac{\delta z}{\delta w^i}$ and $\frac{\delta z}{\delta x^i}$. Using the chain rule, we have

$$\frac{\partial z}{\partial (vec(w^i)^T)} = \frac{\partial z}{\partial (vec(x^{i+1})^T)} \frac{\partial vec\left(x^{i+1}\right)}{\partial (vec(w^i)^T)},$$

$$\frac{\partial z}{\partial (vec(x^i)^T)} = \frac{\partial z}{\partial (vec(x^{i+1})^T)} \frac{\partial vec\left(x^{i+1}\right)}{\partial (vec(x^i)^T)},$$

Since $\frac{\partial z}{\partial x^{i+1}}$ is already computed and stored in memory, it requires just a matrix reshaping operation (vec) and an additional transpose operation to get $\frac{\partial z}{\partial vec(x^{i+1})}$, which is the first term in the right hand side (RHS) of both equations. So long as we can compute $\frac{\partial vec(x^{i+1})}{\partial (vec(w^i)^T)}$ and $\frac{\partial vec(x^{i+1})}{\partial (vec(x^i)^T)}$, we can easily get what we want (the left hand side of both equations). $\frac{\partial vec(x^{i+1})}{\partial (vec(w^i)^T)}$ and $\frac{\partial vec(x^{i+1})}{\partial (vec(x^i)^T)}$ are much easier to compute than directly computing $\frac{\partial z}{\partial (vec(w^i)^T)}$ and $\frac{\partial z}{\partial (vec(x^i)^T)}$, because $x^i$ is directly related to $x^{i+1}$, through a function with parameters $x^i$.

In the context of neural networks, activations serve as transfer functions between the input of a neuron and the output. They define under which conditions the node is activated, i.e. the input values are mapped to the output which, in hidden layers, serves again as one of the inputs to the succeeding neuron. There exists a vast amount of different activation functions with different characteristics.

A loss function quantifies how well an algorithm models the given data. To learn from the data and in order to change the weights of the network, the loss function has to be minimized. Generally, one can make the distinction between a regression loss and classification loss. Classification predicts output from set of finite categorical values (class labels), and regression, on the other hand, deals with prediction a continuous value.

In the following mathematical formulations, the following parameters are defined as:

- n is the number of training examples
- i is the i-th training example in a data set
- $y_i$ is the ground truth label for the i-th training example
- $\hat{y}_i$ is the prediction for i-th training example

The most common setting for classification problems is cross-entropy loss. It increases as the predicted probability diverges from the actual label. The log of the actual predicted probability is multiplied with the ground truth class. An important aspect of this is that cross entropy loss penalizes heavily the predictions that are confident but wrong. The mathematical formulation can be described as:

$$CrossEntropyLoss = -(y_i log(\hat{y}_i) + (1 - y_i) log(1 - \hat{y_i})) \qquad (7)$$

A typical example for a regression loss is the mean square error or $L_2$ loss. As the name suggests, mean square error is measured as the average of the squared difference between predictions and actual observations. It is only concerned with the average magnitude of error irrespective of their direction. However, due to squaring, predictions which are far away from actual values are penalized heavily in comparison to less deviated predictions. Plus MSE has nice mathematical properties which makes it easier to calculate gradients. Its formulation is as follows:

$$MSE = \frac{1}{n} \cdot \sum_{i=1}^{n} (y_i - \hat{y}_i)^2$$

The following documents contain information on the functioning of convolutional neural networks:

I. Goodfellow, Y. Bengio, and A. Courville, "Deep learning, chapter convolutional networks." http://www.deeplearning book.org, 2016.
J. Wu, "Introduction to convolutional neural networks." https://pdfs.semanticscholar.org/450c/a19932fcef1 ca6d0442cbf52fec38fb9d1e5.pdf. "Common loss functions in machine learning." https://towardsdatascience.com/ common-loss-functions-in-machine-learning-46af0ffc4d23. Accessed: 2019-08-22.
Alex Krizhevsky, Ilya Sutskever, and Geoffrey E. Hinton, "Imagenet classification with deep convolutional neural networks." http://papers.nips.cc/paper/4824-imagenet-classification -with-deep-convolutional-neural-networks.pdf.
S. Ren, K. He, R. Girshick, and J. Sun, "Faster r-cnn: Towards real-time object detection with region proposal networks." https://arxiv.org/pdf/1506.01497.pdf.
S.-E. Wei, V. Ramakrishna, T. Kanade, and Y. Sheikh, "Convolutional pose machines." https://arxiv.org/pdf/ 1602.00134.pdf.
Jonathan Long, Evan Shelhamer, and Trevor Darrell, "Fully convolutional networks for semantic segmentation." https://www.cv-foundation.org/openaccess/content_cvpr_2015/papers/Long_Fully_Convolutional_Ne tworks_2015_CVPR_paper.pdf.

## DEFINITIONS

In this section, definitions for specific terminology used in this disclosure are offered which also form part of the present disclosure.

The method in accordance with the invention is for example a computer-implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term computer includes a server resource. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services™. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz®, a product of Brainlab SE. The monitor may also be the monitor of a portable, for example handheld, device such as a smart phone or personal digital assistant or digital media player.

The invention also relates to a computer program comprising instructions which, when on the program is executed by a computer, cause the computer to carry out the method or methods, for example, the steps of the method or methods, described herein and/or to a computer-readable storage medium (for example, a non-transitory computer-readable storage medium) on which the program is stored and/or to a computer comprising said program storage medium and/or to a (physical, for example electrical, for example technically generated) signal wave, for example a digital signal wave, such as an electromagnetic carrier wave carrying information which represents the program, for example the aforementioned program, which for example comprises code means which are adapted to perform any or all of the method steps described herein. The signal wave is in one example a data carrier signal carrying the aforementioned computer program. The invention also relates to a computer comprising at least one processor and/or the aforementioned computer-readable storage medium and for example a memory, wherein the program is executed by the processor.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. A step of "determining" as described herein for example comprises or consists of issuing a command to perform the determination described herein. For example, the step comprises or consists of issuing a command to cause a computer, for example a remote computer, for example a remote server, for example in the cloud, to perform the determination. Alternatively or additionally, a step of "determination" as described herein for example comprises or consists of receiving the data resulting from the determination described herein, for example receiving the resulting data from the remote computer, for example from that remote computer which has been caused to perform the determination. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

Image registration is the process of transforming different sets of data into one coordinate system. The data can be multiple photographs and/or data from different sensors, different times or different viewpoints. It is used in computer vision, medical imaging and in compiling and analysing images and data from satellites. Registration is necessary in order to be able to compare or integrate the data obtained from these different measurements.

Preferably, atlas data is acquired which describes (for example defines, more particularly represents and/or is) a general three-dimensional shape of the anatomical body part. The atlas data therefore represents an atlas of the anatomical body part. An atlas typically consists of a plurality of generic models of objects, wherein the generic models of the objects together form a complex structure. For example, the atlas constitutes a statistical model of a patient's body (for example, a part of the body) which has been generated from anatomic information gathered from a plurality of human bodies, for example from medical image data containing images of such human bodies. In principle, the atlas data therefore represents the result of a statistical analysis of such medical image data for a plurality of human bodies. This result can be output as an image - the atlas data therefore contains or is comparable to medical image data. Such a comparison can be carried out for example by applying an image fusion algorithm which conducts an image fusion between the atlas data and the medical image data. The result of the comparison can be a measure of similarity between the atlas data and the medical image data. The atlas data comprises image information (for example, positional image information) which can be matched (for example by applying an elastic or rigid image fusion algorithm) for example to image information (for example, positional image information) contained in medical image data so as to for example compare the atlas data to the medical image data in order to determine the position of anatomical structures in the medical image data which correspond to anatomical structures defined by the atlas data.

The human bodies, the anatomy of which serves as an input for generating the atlas data, advantageously share a common feature such as at least one of gender, age, ethnicity, body measurements (e.g. size and/or mass) and pathologic state. The anatomic information describes for example the anatomy of the human bodies and is extracted for example from medical image information about the human bodies. The atlas of a femur, for example, can comprise the head, the neck, the body, the greater trochanter, the lesser trochanter and the lower extremity as objects which together make up the complete structure. The atlas of a brain, for example, can comprise the telencephalon, the cerebellum, the diencephalon, the pons, the mesencephalon and the medulla as the objects which together make up the complex structure. One application of such an atlas is in the segmentation of medical images, in which the atlas is matched to medical image data, and the image data are compared with the matched atlas in order to assign a point (a pixel or voxel) of the image data to an object of the matched atlas, thereby segmenting the image data into objects.

For example, the atlas data includes information of the anatomical body part. This information is for example at least one of patient-specific, non-patient-specific, indication-specific or non-indication-specific. The atlas data therefore describes for example at least one of a patient-specific, non-patient-specific, indication-specific or non-indication-specific atlas. For example, the atlas data includes movement information indicating a degree of freedom of movement of the anatomical body part with respect to a given reference (e.g. another anatomical body part). For example, the atlas is a multimodal atlas which defines atlas information for a plurality of (i.e. at least two) imaging modalities and contains a mapping between the atlas information in different imaging modalities (for example, a mapping between all of the modalities) so that the atlas can be used for transforming medical image information from its image depiction in a first imaging modality into its image depiction in a second imaging modality which is different from the first imaging modality or to compare (for example, match or register) images of different imaging modality with one another.

The present invention relates to the field of controlling a treatment beam. The treatment beam treats body parts which are to be treated and which are referred to in the following as "treatment body parts". These body parts are for example parts of a patient's body, i.e. anatomical body parts.

The present invention relates to the field of medicine and for example to the use of beams, such as radiation beams, to treat parts of a patient's body, which are therefore also referred to as treatment beams. A treatment beam treats body parts which are to be treated and which are referred to in the following as "treatment body parts". These body parts are for example parts of a patient's body, i.e. anatomical body parts. Ionising radiation is for example used for the purpose of treatment. For example, the treatment beam comprises or consists of ionising radiation. The ionising radiation comprises or consists of particles (for example, sub-atomic particles or ions) or electromagnetic waves which are energetic enough to detach electrons from atoms or molecules and so ionise them. Examples of such ionising radiation include X-rays, high-energy particles (high-energy particle beams) and/or ionising radiation emitted from a radioactive element. The treatment radiation, for example the treatment beam, is for example used in radiation therapy or radiotherapy, such as in the field of oncology. For treating cancer in particular, parts of the body comprising a pathological structure or tissue such as a tumour are treated using ionising radiation. The tumour is then an example of a treatment body part.

The treatment beam is preferably controlled such that it passes through the treatment body part. However, the treatment beam can have a negative effect on body parts outside the treatment body part. These body parts are referred to here as "outside body parts". Generally, a treatment beam has to pass through outside body parts in order to reach and so pass through the treatment body part.

Reference is also made in this respect to the following web pages: http://www.elekta.com/healthcare_us_elek ta_vmat.php and http://www.varian.com/us/oncology/treatments/treatment_techniques/rapidarc.

In the field of medicine, imaging methods (also called imaging modalities and/or medical imaging modalities) are used to generate image data (for example, two-dimensional or three-dimensional image data) of anatomical structures (such as soft tissues, bones, organs, etc.) of the human body. The term "medical imaging methods" is understood to mean (advantageously apparatus-based) imaging methods (for example so-called medical imaging modalities and/or radiological imaging methods) such as for instance computed tomography (CT) and cone beam computed tomography (CBCT, such as volumetric CBCT), X-ray tomography, magnetic resonance tomography (MRT or MRI), conventional X-ray, sonography and/or ultrasound examinations, and positron emission tomography. For example, the medical imaging methods are performed by the analytical devices. Examples for medical imaging modalities applied by medical imaging methods are: X-ray radiography, magnetic resonance imaging, medical ultrasonography or ultrasound, endoscopy, elastography, tactile imaging, thermography, medical photography and nuclear medicine functional imaging techniques as positron emission tomography (PET) and Single-photon emission computed tomography (SPECT), as mentioned by Wikipedia. The image data thus generated is also termed "medical imaging data". Analytical devices for example are used to generate the image data in apparatus-based imaging methods. The imaging methods are for example used for medical diagnostics, to analyse the anatomical body in order to generate images which are described by the image data. The imaging methods are also for example used to detect pathological changes in the human body. However, some of the changes in the anatomical structure, such as the pathological changes in the structures (tissue), may not be detectable and for example may not be visible in the images generated by the imaging methods. A tumour represents an example of a change in an anatomical structure. If the tumour grows, it may then be said to represent an expanded anatomical structure.

This expanded anatomical structure may not be detectable; for example, only a part of the expanded anatomical structure may be detectable. Primary/high-grade brain tumours are for example usually visible on MRI scans when contrast agents are used to infiltrate the tumour. MRI scans represent an example of an imaging method. In the case of MRI scans of such brain tumours, the signal enhancement in the MRI images (due to the contrast agents infiltrating the tumour) is considered to represent the solid tumour mass. Thus, the tumour is detectable and for example discernible in the image generated by the imaging method. In addition to these tumours, referred to as "enhancing" tumours, it is thought that approximately 10% of brain tumours are not discernible on a scan and are for example not visible to a user looking at the images generated by the imaging method.

Mapping describes a transformation (for example, linear transformation) of an element (for example, a pixel or voxel), for example the position of an element, of a first data set in a first coordinate system to an element (for example, a pixel or voxel), for example the position of an element, of a second data set in a second coordinate system (which may have a basis which is different from the basis of the first coordinate system). In one embodiment, the mapping is determined by comparing (for example, matching) the color values (for example grey values) of the respective elements by means of an elastic or rigid fusion algorithm. The mapping is embodied for example by a transformation matrix (such as a matrix defining an affine transformation).

Image fusion can be elastic image fusion or rigid image fusion. In the case of rigid image fusion, the relative position between the pixels of a 2D image and/or voxels of a 3D image is fixed, while in the case of elastic image fusion, the relative positions are allowed to change.

In this application, the term "image morphing" is also used as an alternative to the term "elastic image fusion", but with the same meaning.

Elastic fusion transformations (for example, elastic image fusion transformations) are for example designed to enable a seamless transition from one dataset (for example a first dataset such as for example a first image) to another dataset (for example a second dataset such as for example a second image). The transformation is for example designed such that one of the first and second datasets (images) is deformed, for example in such a way that corresponding structures (for example, corresponding image elements) are arranged at the same position as in the other of the first and second images. The deformed (transformed) image which is transformed from one of the first and second images is for example as similar as possible to the other of the first and second images. Preferably, (numerical) optimisation algorithms are applied in order to find the transformation which results in an optimum degree of similarity. The degree of similarity is preferably measured by way of a measure of similarity (also referred to in the following as a "similarity measure"). The parameters of the optimisation algorithm are for example vectors of a deformation field. These vectors are determined by the optimisation algorithm in such a way as to result in an optimum degree of similarity. Thus, the optimum degree of similarity represents a condition, for example a constraint, for the optimisation algorithm. The bases of the vectors lie for example at voxel positions of one of the first and second images which is to be transformed, and the tips of the vectors lie at the corresponding voxel positions in the transformed image. A plurality of these vectors is preferably provided, for instance more than twenty or a hundred or a thousand or ten thousand, etc. Preferably, there are (other) constraints on the transformation (deformation), for example in order to avoid pathological deformations (for instance, all the voxels being shifted to the same position by the transformation). These constraints include for example the constraint that the transformation is regular, which for example means that a Jacobian determinant calculated from a matrix of the deformation field (for example, the vector field) is larger than zero, and also the constraint that the transformed (deformed) image is not self-intersecting and for example that the transformed (deformed) image does not comprise faults and/or ruptures. The constraints include for example the constraint that if a regular grid is transformed simultaneously with the image and in a corresponding manner, the grid is not allowed to interfold at any of its locations. The optimising problem is for example solved iteratively, for example by means of an optimisation algorithm which is for example a first-order optimisation algorithm, such as a gradient descent algorithm. Other examples of optimisation algorithms include optimisation algorithms which do not use derivations, such as the downhill simplex algorithm, or algorithms which use higher-order derivatives such as Newton-like algorithms. The optimisation algorithm preferably performs a local optimisation. If there is a plurality of local optima, global algorithms such as simulated annealing or generic algorithms can be used. In the case of linear optimisation problems, the simplex method can for instance be used.

In the steps of the optimisation algorithms, the voxels are for example shifted by a magnitude in a direction such that the degree of similarity is increased. This magnitude is preferably less than a predefined limit, for instance less than one tenth or one hundredth or one thousandth of the diameter of the image, and for example about equal to or less than the distance between neighbouring voxels. Large deformations can be implemented, for example due to a high number of (iteration) steps.

The determined elastic fusion transformation can for example be used to determine a degree of similarity (or similarity measure, see above) between the first and second datasets (first and second images). To this end, the deviation between the elastic fusion transformation and an identity transformation is determined. The degree of deviation can for instance be calculated by determining the difference between the determinant of the elastic fusion transformation and the identity transformation. The higher the deviation, the lower the similarity, hence the degree of deviation can be used to determine a measure of similarity.

A measure of similarity can for example be determined on the basis of a determined correlation between the first and second datasets.

**BRIEF DESCRIPTION OF THE DRAWINGS**

In the following, the invention is described with reference to the appended figures which give background explanations and represent specific embodiments of the invention. The scope of the invention is however not limited to the specific features disclosed in the context of the figures, wherein

Fig. 1      illustrates the basic steps of the method according to the first aspect;
Fig. 2      is a flow diagram showing an example of the method according to the first aspect;
Fig. 3      is a flow diagram showing an example of the method according to the first aspect;
Fig. 4      is a flow diagram showing an example of the method according to the first or second aspect;
Fig. 5      is a flow diagram showing an example of the method according to the second aspect;
Fig. 6      is a flow diagram showing an example of the method according to the second aspect;
Fig. 7      illustrates the basic steps of the method according to the second aspect;
Fig. 8      is a schematic illustration of the functionality of a neuron of a convolutional neural network usable to implement the method according to the second aspect;
Fig. 9      shows an example of the methods according to the first, second and third aspects;
Fig. 10    is a schematic illustration of the system according to the seventh aspect.

**DESCRIPTION OF EMBODIMENTS**

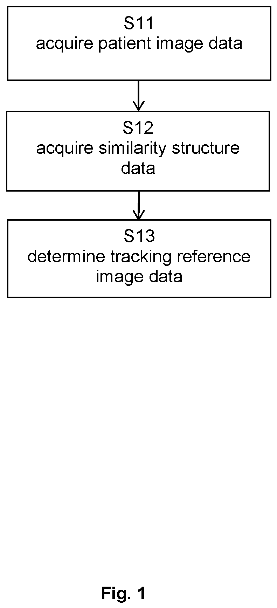

Fig. 1 illustrates the basic steps of the method according to the first aspect, in which step S11 encompasses acquisition of the patient image data, step S12 encompasses acquisition of the similarity structure data and subsequent step S13 encompasses determination of the tracking reference image data.

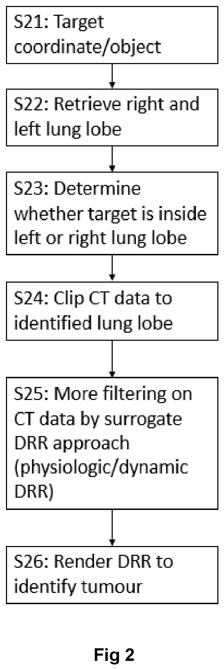

Fig. 2 shows an embodiment of the method according to the first aspect in which step S21 is directed to determining, from the planning image, a data object describing the target and the image coordinates of that data object. In this example, the anatomical body part is the lung Step S22 continues with retrieving the data objects and their image coordinates describing the right and left lobes of the lung. In step S23, it is then determined whether the target is located in the left or right lobe. Step S24 clips the CT data, i.e.t the planning image to the identified left or right lobe. This means that only the data object defining the identified lobe is output from step S24. In step S25, The CT data is filtered by applying the surrogate DRR approach disclosed in US 10,776,959 B2. Thereby, a series of dynamic DRRs is generated which shows the possible movement states of the target. At least one such DRR is then rendered to identify the position of the tumour.

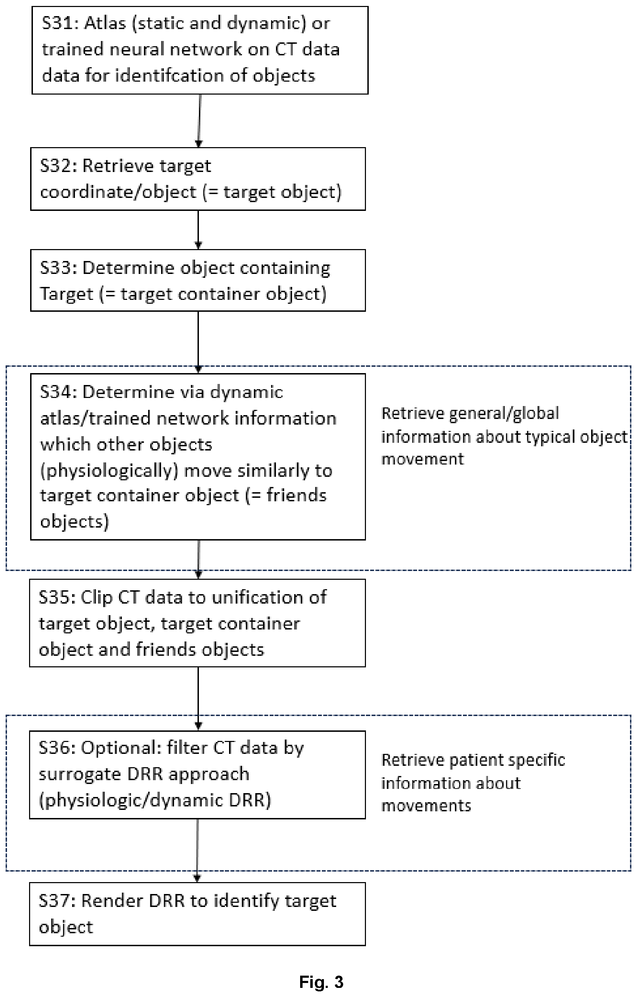

Fig. 3 shows an example of the method according to the first aspect which is directed to using an atlas comprising i.a. dynamic atlas data, called dynamic atlas, or a an appropriately trained neural network for identification of the anatomical similarity structure. In step S31, an atlas comprising only static atlas data or static atlas data and dynamic atlas data or an appropriately trained neural network (which has been trained for example by applying the method according to the second aspect) is used to identify objects in the planning image. In step S32, a data object describing the target is determined as a target object by retrieving its image coordinates in the planning image. In step S33, a data object containing the target object is determined as a target container object. For example, a data object describing a lung lobe is determined as a target container object comprising a tumour as the target object which is located in the respective lung lobe. In steps S34, the atlas or the trained neural network is used to determine, from the data objects identified in step S31, objects which physiologically move similarly to the target container object. Such objects are called friends objects. Step S34 corresponds to retrieving general (global) information about typical object movement for the patient under consideration. Step S35 then continues with clipping the planning image to a unification of the target object, the target container object and the friends objects. In optional step S36, filtering is performed on the planning image by applying the surrogate DRR approach disclosed in US 10,776,959 B2. This serves retrieval of patient-specific information about movement of anatomical structures. In step S37, a DRR is rendered from the planning image to identify the target object.

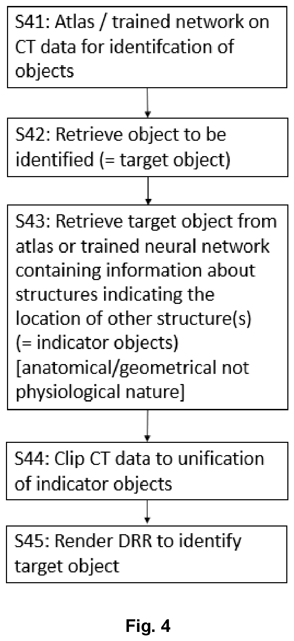

Fig. 4 shows an example of the method according to the first aspect which is directed to using, in step S41, an atlas comprising dynamic atlas data, called dynamic atlas, or an appropriately trained neural network for identification of the anatomical similarity structure for identification of data objects in the planning image. In step S42, a data object describing the target is determined as a target object by retrieving its image coordinates in the planning image. Step S43 then retrieves the target objects using the atlas which contains information about anatomical structures indicating the location of another anatomical structure or other anatomical structures, called indicator objects. The atlas is matched with the planning image. The location of the other anatomical structure or other anatomical structures is deduced from the anatomical (for example geometrical) nature of the anatomical structures rather than their physiological function.

Alternatively, this retrieval is made by running an appropriately trained neural network on the planning image. In step S44, the planning image is then clipped to a unification of the indicator objects. In step S45, a DRR is rendered from the planning image to identify the target object. Examples of movement information include information about similar movement of the bronchi or the diaphragm in the case of the target being a lung tumour; the head or the lower jaws which can move relative to the skull; the spinal cord which has a movement similar to the vertrebrae surrounding it; or and, as an example of a positionally inert structure, a fixed position of an anatomical structure relative to the target (for example, if the target adheres to the indicator object).

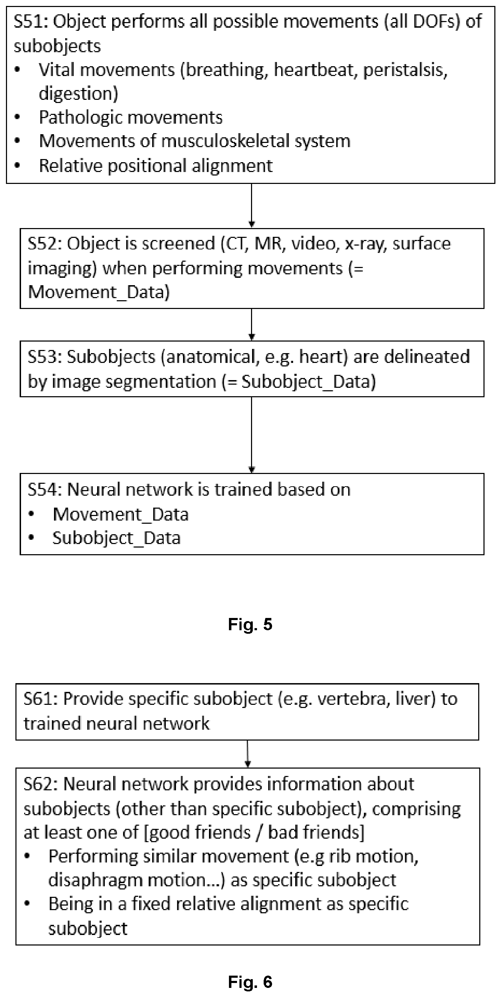

Fig. 5 is a flow diagram showing an example of the method according to the second aspect with regard to training a neural network. In step S51, image training data is acquired which describes data objects comprising subobjects which perform all possible movements in all degrees of freedom (DOFs). These movements are due to for example vital movements such as breathing, heartbeat, peristalsis or digestion, or are pathologic movements or movements of the muscoskeletal system or due to a change of the positional alignment of the patient relative to the radiation treatment device. The object is screened in step S52 when it moves, for example by CT (computed x-ray tomography) or MR (magnetic resonance) imaging, video imaging or x-ray (radiographic) imaging. The output of this step is called Movement_Data. In steps S53, the subobjects (which constitute anatomical subobjects such as the heart) are delineated by image segmentation. The output of this step is called Subobject_Data. In step S54, the neural network is trained based on Movement_Data and Subobject_Data to determine anatomical structures having a movement pattern which is similar to a movement pattern of the target.

Fig. 6 illustrates deployment, i.e. use, of the trained neural network generated by the method according to the second aspect. In step S61, a specific subobject representing a vertebra or the liver is provided to the trained neural network, and the network then in step S62 provides information about subobjects other than the specific subobject which can include good friends (i.e. subobjects having a movement pattern which is similar to the movement pattern of the target) and bad friends (i.e. subobjects having a movement pattern which is dissimilar to the movement pattern of the target). The movement pattern of a friend is determined to be similar to that of the target for example if the friend performs a movement (e.g. rib motion or diaphragm motion) which is similar to the motion of the subobject, or if a friend is in a fixed position relative to the specific subobject (for example, if the specific subobject, i.e. the target, adheres to the other subobject).

Fig. 7 illustrates the basic steps of the method according to the second aspect, in which step S71 encompasses acquisition of the image training data, and subsequent step S72 encompasses determination of the similarity structure data.

Fig. 8 is a schematic explanation of an artificial neuron which constitutes a main component of convolutional neural network. The figure shows an example of a single neuron. The node in the middle represents a neuron, which takes all inputs and multiplies them with their specific weights. The importance of the input depends on the value of its weight. The addition of these computed values is called weighted sum which will be inserted into an activation function. The bias is an input-independent value which modifies the boundaries of the threshold. The resulting value is processed by an activation function which decides whether the input will be transferred to the next neuron.

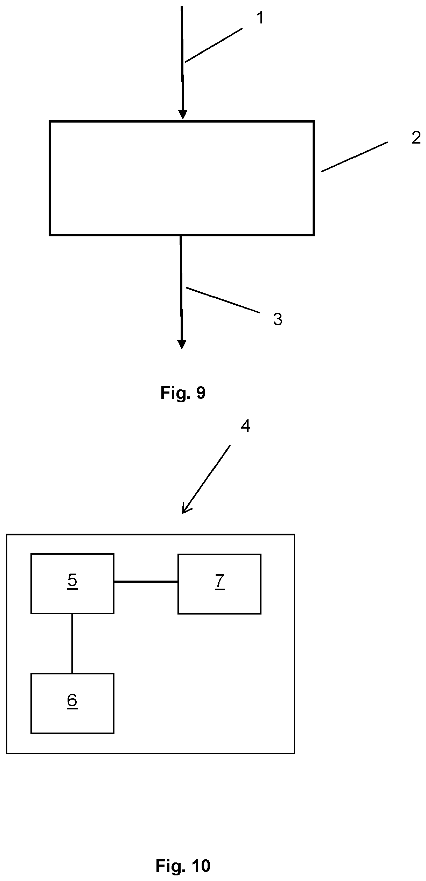

Fig. 9 illustrates an embodiment of the present invention that includes all essential features of the invention. In this embodiment, the entire data processing which is part of the method according to the first, second or third aspect is performed by a computer 2. Reference sign 1 denotes the input of data acquired by the method according to the first, second or third aspect into the computer 2 and reference sign 3 denotes the output of data determined by the method according to the first, second or third aspect.

Fig. 10 is a schematic illustration of the medical system 4 according to the seventh aspect. The system is in its entirety identified by reference sign 4 and comprises a computer 5, a (for example non-transitory) program storage medium 8, a (for example non-transitory) electronic data storage device 6 for storing at least the patient data and a medical device 7 (such as a radiation treatment device). The components of the medical system 4 have the functionalities and properties explained above with regard to the seventh aspect of this disclosure.

The invention relates to the following embodiments A to Y:

A. A computer-implemented method of determining a digital image usable for tracking the position of a target structure in radiation treatment, the method comprising the following steps:

a) patient image data is acquired (S11) which describes a digital patient image including an image representation of an anatomical body part of a patient including an image representation of the target structure, wherein the target structure is associated with a target movement pattern describing a sequence of positions of the target structure;
b) similarity structure data is acquired (S12) which describes an anatomical similarity structure included in the anatomical body part which is associated with a similar movement pattern which is similar to the target movement pattern, the similar movement pattern describing a sequence of positions of the anatomical similarity structure, wherein the patient image includes an image representation of the anatomical similarity structure;
c) tracking reference image data is determined (S13) based on the patient image data and the similarity structure data, wherein the tracking reference image data describes a digital tracking reference image including an image representation of the target structure and an image representation of the anatomical similarity structure, wherein the tracking reference image is determined by extracting, from the patient image, the image representation of the target structure and the image representation of the anatomical similarity structure from the patient image.

B. The method according to the preceding embodiment, wherein the tracking image data is determined by extracting, from the patient image, a data object including the image representation of the target structure and a data object including the anatomical similarity structure.

C. The method according to any one of the preceding embodiments, comprising

acquiring atlas data describing a digital model of the anatomical body part, wherein the atlas data comprises static atlas data describing data objects, called atlas objects, defining a spatial property of constituents of the anatomical body part;
acquiring target position data which describes a position of the target structure in the patient image;
determining, based on the target position data and the atlas data, target object data describing a data object in the atlas data including the target structure, called atlas target object;
determining, based on the target object data and the patient image data, the similarity structure data.

D. The method according to the preceding embodiment,

wherein the atlas data comprises dynamic atlas data describing a dynamic property for each of the data objects;
determining, based on the target object data, target property data describing the dynamic property associated with the target data object, called target property;
determining, based on the target property data and the atlas data, similarity object data describing a data object, called atlas similarity object, associated with a dynamic property which is similar to the target property, called similarity property;
determining, based on the similarity object data and the patient image data, the similarity structure data.

E. The method according to the preceding embodiment, wherein the image representation of the target structure is included in a data object in the patient image called patient target object and the image representation of the anatomical similarity structure is included in a data object in the patient image called patient similarity object, and the tracking image data is determined by extracting the patient target object and the similarity object from the patient image.

F. The method according to any one of the preceding embodiments, wherein the dynamic property of the target data object described by the dynamic atlas data is the target movement pattern and the dynamic property of the similarity data object described by the dynamic atlas data is the similarity movement pattern.

G. The method according to any one of the preceding embodiments, wherein the wherein the dynamic property describes a change of the spatial property of the associated data object, for example at least one of a change of position of the associated atlas object and a change of geometry of the associated atlas object.

H. The method according to any one of the preceding embodiments, wherein the dynamic property describes at least one trajectory defining a time-dependent position of the associated atlas object or of a point with a known spatial relationship to the associated atlas object, such as a centre of the associated atlas object or a point on the outline of the associated atlas object.

I. The method according to any one of the preceding embodiments, wherein the dynamic property describes correlations between the dynamic properties associated with different ones of the atlas objects, for example correlations between different trajectories of associated atlas objects.

J. The method according to any one of the preceding embodiments, wherein the dynamic property classified according to patient type.

K. The method according to any one of the preceding embodiments, wherein the dynamic property is used as a constraint for matching the digital model with the patient image, for example to determine at least one of the similarity object data and the similarity structure data.

L. A computer-implemented method of training a trainable algorithm to determine anatomical structures having a similar movement pattern, the method comprising the following steps:

a) image training data (S71) is acquired which describes at least two digital training images of an anatomical body part including a training target data object including an image representation of an anatomical body part of a patient, the image representation of the anatomical body part including an image representation of a target structure, wherein the target structure is associated with a training target movement pattern describing a sequence of positions of the target structure, wherein the training images have been generated at different points in time and describe different positions of the of the target structure, wherein the training images include at least one similarity training data object including an image representation of a training similarity structure included in the anatomical body part, the training similarity structure being associated with a training similar movement pattern which is similar to the target movement pattern, the similar movement pattern describing a sequence of positions of the training similarity structure;

b) similarity structure data is determined (S72) which describes model parameters of a trainable algorithm for establishing a relation between the target structure and the training similarity structure, wherein the relation describes whether the training similar movement pattern is similar to the training target movement pattern and is determined by inputting the image training data into a function which establishes the relation.

M. The method according to the preceding embodiment, comprising

acquiring similarity training data which describes whether the training target movement pattern is similar to the training similar movement pattern,
wherein the similarity structure data is determined by inputting the similarity training data into the function.

N. The method according to the preceding embodiment, wherein the similarity structure data is determined by determining whether the relation fulfils a predetermined criterion which defines that the training similar movement pattern is similar to the training target movement pattern, by comparing the relationship to the predetermined criterion and determining that the relationship fulfils the predetermined criterion.

O. The method according to any one of the three immediately preceding embodiments,
wherein the medical image training data is patient image data or has been generated from atlas data describing a digital model of the anatomical body part.

P. The method according to any one of the preceding embodiments, wherein the similarity structure data is determined by inputting the patient image data into a trainable algorithm configured to determine the similarity structure data.

Q. The method according to the preceding embodiment, wherein the trainable algorithm has been trained by executing the method according to any one of the preceding embodiments.

R. The method according to any one of the preceding embodiments, wherein the trainable algorithm comprises or consists of a learning algorithm, for example a machine learning algorithm.

S. The method according to any one of the preceding embodiments, wherein the trainable algorithm comprises or consists of a convolutional neural network.

T. The method according to any one of the preceding embodiments, wherein the model parameters of the trainable algorithm define the trainable parameters, for example weights, of the trainable algorithm.

U. A computer-implemented method of determining control data for controlling the operation of a radiation treatment device, the method comprising the following steps:

a) executing the method according to any one of the preceding embodiments;
b) acquiring tracking patient image data describing a digital patient tracking image including an image representation of the anatomical similarity structure generated by imaging the anatomical body part;
c) determining, based on the tracking reference image data and the tracking patient image data, control data for controlling the operation of a radiation treatment device.

V. The method according to the preceding embodiment, wherein the tracking reference image and the tracking patient image are spatially comparable, for example positions in the tracking reference image and the tracking patient image are defined in a common reference system or related to each other by a known spatial transformation, and wherein the control data is determined by comparing the position of the image representation of the similarity structure in the tracking reference image to the position of the image representation of the similarity structure in the tracking patient image.

W. The method according to any one of the preceding embodiments, comprising controlling the radiation treatment device based on the control data, for example by executing the control data.

X. A program which, when running on a computer (2) or when loaded onto a computer (2), causes the computer (2) to perform the method steps of the method according to any one of the preceding claims, and/or a program storage medium on which the program is stored or a program storage medium on which data defining the model parameters and the architecture of a trainable algorithm which has been trained by executing the method according to any one of embodiments L to O or R to T as far as dependent on any one of embodiments L to O are stored, and/or a data carrier signal carrying the aforementioned program, and/ or a data carrier signal carrying data defining the model parameters and the architecture of a trainable algorithm which has been trained by executing the method according to any one of embodiments L to O or R to T as far as dependent on any one of embodiments L to O, and/or a data stream which carries the aforementioned program, and/or a data stream which carries data defining the model parameters and the architecture of a learning algorithm which has been trained by executing the method according to any one of embodiments L to O or R to T as far as dependent on any one of embodiments L to O, and/or at least one computer (2) comprising at least one processor and a memory, wherein the aforementioned program is running on the at least one processor or loaded into the memory of the computer (2).

Y. A system for planning or conducting radiation treatment (1), comprising:

a) the at least one computer (2) according to the preceding embodiment;
b) at least one electronic data storage device (3) storing the patient image data; and
c) the program storage medium according to the preceding embodiment,
wherein the at least one computer (2) is operably coupled to the at least one electronic data storage device (3) for acquiring, from the at least one electronic data storage device (3), the patient image data, and for storing, in the at least one electronic data storage device (3), at least the tracking reference image data.

**Claims**

1. A computer-implemented method of training a trainable algorithm to determine anatomical structures having a similar movement pattern, the method comprising the following steps:

a) image training data (S71) is acquired which describes at least two digital training images of an anatomical body part including a training target data object including an image representation of an anatomical body part of a patient, the image representation of the anatomical body part including an image representation of a target structure, wherein the target structure is associated with a training target movement pattern describing a sequence of positions of the target structure, wherein the training images have been generated at different points in time and describe different positions of the of the target structure, wherein the training images include at least one similarity training data object including an image representation of a training similarity structure included in the anatomical body part, the training similarity structure being associated with a training similar movement pattern, the similar movement pattern describing a sequence of positions of the training similarity structure;
b) acquiring similarity training data which describes whether the training target movement pattern is similar to the training similar movement pattern;
c) similarity structure data is determined (S72) which describes model parameters of a trainable algorithm for establishing a relation between the target structure and the training similarity structure, wherein the relation describes whether the training similar movement pattern is similar to the training target movement pattern and is determined by inputting the image training data and the similarity training data into a function which establishes the relation.

2. The method according to claim 1, wherein the similarity structure data is determined by determining whether the relation fulfils a predetermined criterion which defines that the training similar movement pattern is similar to the training target movement pattern, by comparing the relationship to the predetermined criterion and determining that the relationship fulfils the predetermined criterion.

3. The method according to any one of the two immediately preceding claims, wherein the image training data is patient image data or has been generated from atlas data describing a digital model of the anatomical body part.

4. A computer-implemented method of determining a digital image usable for tracking the position of a target structure in radiation treatment, the method comprising the following steps:

a) patient image data is acquired (S11) which describes a three-dimensional digital patient image including an image representation of an anatomical body part of a patient including an image representation of the target structure, wherein the target structure is a target of radiation treatment and associated with a target movement pattern describing a sequence of positions of the target structure, wherein the patient image data is time-dependent and is or has been generated to describe the position of the target structure at different points in time;
b) similarity structure data is acquired (S12) which describes an anatomical similarity structure included in the anatomical body part which is associated with a similar movement pattern which is similar to the target movement pattern, the similar movement pattern describing a sequence of positions of the anatomical similarity structure, wherein the patient image includes an image representation of the anatomical similarity structure;
c) tracking reference image data is determined (S13) based on the patient image data and the similarity structure data, wherein the tracking reference image data describes a digital tracking reference image including an image representation of the target structure and an image representation of the anatomical similarity structure, wherein the tracking reference image is determined by extracting, from the patient image, a synthesized two-dimensional medical image containing the image representation of the target structure and the image representation of the anatomical similarity structure,

**characterized in that**
the similarity structure data is acquired by executing a trainable algorithm configured to determine the similarity structure data.

5. The method according to the preceding claim, wherein the tracking image data is determined by extracting, from the patient image, a data object including the image representation of the target structure and a data object including the anatomical similarity structure.

6. A computer-implemented method of determining control data for controlling the operation of a radiation treatment device, the method comprising the following steps:

a) executing the method according to any one claims 4 to 5;
b) acquiring tracking patient image data describing a digital patient tracking image including an image representation of the anatomical similarity structure generated by imaging the anatomical body part;
c) determining, based on the tracking reference image data and the tracking patient image data, control data for controlling the operation of a radiation treatment device, wherein the control data is determined by comparing the position of the image representation of the anatomical similarity structure in the tracking reference image to the position of the image representation of the anatomical similarity structure in the tracking patient image and generating a control signal for controlling the operation of the radiation treatment device if the result of the comparison fulfills a predetermined condition.

7. A program which, when running on a computer (2) or when loaded onto a computer (2), causes the computer (2) to perform the method steps of the method according to any one of the preceding claims.

8. A program storage medium on which the program according to claim 7 is stored or a program storage medium on which data defining the model parameters and the architecture of a trainable algorithm which has been trained by executing the method according to any one of claims 1 to 3 are stored.

9. A data carrier signal carrying the aforementioned program according to claim 7, and/ or a data carrier signal carrying data defining the model parameters and the architecture of a trainable algorithm which has been trained by executing the method according to any one of claims 1 to 3.

10. At least one computer (2) comprising at least one processor and a memory, wherein the program according to claim 7 is running on the at least one processor or loaded into the memory of the computer (2).

**11.** A system for planning or conducting radiation treatment (1), comprising:

a) the at least one computer (2) according to the preceding claim, as far as the program, when running on the computer (2) or when loaded onto the computer (2), causes the computer (2) to perform the steps of the method according to any one of claims 4 to 5;
b) at least one electronic data storage device (3) storing the patient image data; and
c) the program storage medium according to the preceding claim,
wherein the at least one computer (2) is operably coupled to the at least one electronic data storage device (3) for acquiring, from the at least one electronic data storage device (3), the patient image data, and for storing, in the at least one electronic data storage device (3), at least the tracking reference image data.

S11
acquire patient image data

S12
acquire similarity structure
data

S13
determine tracking reference
image data

**Fig. 1**

S21: Target coordinate/object

↓

S22: Retrieve right and left lung lobe

↓

S23: Determine whether target is inside left or right lung lobe

↓

S24: Clip CT data to identified lung lobe

↓

S25: More filtering on CT data by surrogate DRR approach (physiologic/dynamic DRR)

↓

S26: Render DRR to identify tumour

**Fig 2**

S31: Atlas (static and dynamic) or trained neural network on CT data data for identifcation of objects

↓

S32: Retrieve target coordinate/object (= target object)

↓

S33: Determine object containing Target (= target container object)

↓

S34: Determine via dynamic atlas/trained network information which other objects (physiologically) move similarly to target container object (= friends objects)

Retrieve general/global information about typical object movement

↓

S35: Clip CT data to unification of target object, target container object and friends objects

↓

S36: Optional: filter CT data by surrogate DRR approach (physiologic/dynamic DRR)

Retrieve patient specific information about movements

↓

S37: Render DRR to identify target object

**Fig. 3**

S41: Atlas / trained network on CT data for identifcation of objects

S42: Retrieve object to be identified (= target object)

S43: Retrieve target object from atlas or trained neural network containing information about structures indicating the location of other structure(s) (= indicator objects) [anatomical/geometrical not physiological nature]

S44: Clip CT data to unification of indicator objects

S45: Render DRR to identify target object

**Fig. 4**

S51: Object performs all possible movements (all DOFs) of subobjects
- Vital movements (breathing, heartbeat, peristalsis, digestion)
- Pathologic movements
- Movements of musculoskeletal system
- Relative positional alignment

S52: Object is screened (CT, MR, video, x-ray, surface imaging) when performing movements (= Movement_Data)

S53: Subobjects (anatomical, e.g. heart) are delineated by image segmentation (= Subobject_Data)

S54: Neural network is trained based on
- Movement_Data
- Subobject_Data

**Fig. 5**

S61: Provide specific subobject (e.g. vertebra, liver) to trained neural network

S62: Neural network provides information about subobjects (other than specific subobject), comprising at least one of [good friends / bad friends]
- Performing similar movement (e.g rib motion, disaphragm motion...) as specific subobject
- Being in a fixed relative alignment as specific subobject

**Fig. 6**

S71
acquire image training data

S72
determine similarity structure
data

**Fig. 7**

A single neuron

input ✕ weight

input ✕ weight

input ✕ weight

input ✕ weight

bias

Activation
function

Output

**Fig. 8**

**Fig. 9**

**Fig. 10**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10776959 B2

**Non-patent literature cited in the description**

- **I. GOODFELLOW** ; **Y. BENGIO** ; **A. COURVILLE**. *Deep learning, chapter convolutional networks*, 2016, http://www.deeplearningbook.org
- **J. WU**. Common loss functions in machine learning. *Introduction to convolutional neural networks*, 22 August 2019, https://towardsdatascience.com/common-loss-functions-in-machine-learning-46af0ffc4d23
- **ALEX KRIZHEVSKY** ; **ILYA SUTSKEVER** ; **GEOFFREY E. HINTON**. *Imagenet classification with deep convolutional neural networks*, http://papers.nips.cc/paper/4824-imagenet-classification -with-deep-convolutional-neural-networks.pdf
- **S. REN** ; **K. HE** ; **R. GIRSHICK** ; **J. SUN**. *Faster r-cnn: Towards real-time object detection with region proposal networks*, https://arxiv.org/pdf/1506.01497.pdf
- **S.-E. WEI** ; **V. RAMAKRISHNA** ; **T. KANADE** ; **Y. SHEIKH**. *Convolutional pose machines*, https://arxiv.org/pdf/1602.00134.pdf
- **JONATHAN LONG** ; **EVAN SHELHAMER** ; **TREVOR DARRELL**. *Fully convolutional networks for semantic segmentation.*, https://www.cv-foundation.org/openaccess/content_cvpr_2015/papers/Long_-Fully_Convolutional_Ne tworks_2015_CVPR_paper.pdf